# EUROPEAN PATENT APPLICATION

(11) **EP 4 523 716 A1**
(43) Date of publication of application: **19.03.2025**
(21) Application number: 23830932.2
(22) Date of filing: 29.05.2023
(51) Int. Cl.: A61M 1/16

(54) **BLOOD PURIFICATION DEVICE, AND METHOD FOR DETERMINING DETECTION DEFECT IN FLOW METER**

(30) Priority: 28.06.2022 JP 2022103783
(71) Applicant: Nikkiso Co., Ltd., Tokyo 150-6022 (JP)
(72) Inventor: KATAYAMA, Yuki, Makinohara-shi Shizuoka 421-0496 (JP)
(74) Representative: Grosse Schumacher Knauer von Hirschhausen
(86) International application number: PCT/JP2023/019909
(87) International publication number: WO 2024/004482

(57) **Abstract**

The present invention comprises: a dialysate supply channel (52); a waste liquid discharge channel (53); a supply-side control flow meter (64) and a supply-side protective flow meter (65) for detecting the flow rate of the dialysate supply channel (52); a discharge-side control flow meter (73) and a discharge-side protective flow meter (74) for detecting the flow rate in the waste liquid discharge channel (53); a first water removal amount calculation unit (101) for calculating a control-side cumulative water removal amount on the basis of the flow rate detected by the supply-side control flow meter (64) and the flow rate detected by the discharge-side control flow meter (73); a second water removal amount calculation unit (102) for calculating a protection-side cumulative water removal amount on the basis of the flow rate detected by the supply-side protective flow meter (65) and the flow rate detected by the discharge-side protective flow meter (74); and a first detection defect determining unit (104) for determining a detection defect in the control flow meters (64), (73) on the basis of the difference between the control-side cumulative water removal amount and the protection-side cumulative water removal amount.

## Description

### TECHNICAL FIELD

The present invention relates to a blood purification device and a method for determining detection defect in flow meter.

### BACKGROUND OF THE INVENTION

There is a blood purification device in which two flow meters are provided on a dialysate circuit on the supply side and the discharge side and the amount of water removed from blood in a dialyzer is controlled by the two flow meters (see Patent Literature 1). This blood purification device (the blood treatment apparatus) includes a dialyzer, a supply-side dialysate line to supply dialysate to the dialyzer, a first pump and a supply-side flow meter (a supply-side flow sensor) that are arranged on the supply-side dialysate line, a discharge-side dialysate line to discharge waste liquid from the dialyzer, and a second pump and a discharge-side flow meter (a discharge-side flow sensor) that are arranged on the discharge-side dialysate line. In this blood purification device, the balance between the flow rate of the supplied liquid and the flow rate of the discharged liquid is controlled by driving the first pump based on a detection result (a volumetric flow rate) of the supply-side flow meter and the second pump based on a detection result (a volumetric flow rate) of the discharge-side flow meter. The amount of water removed from blood in the dialyzer is thereby controlled.

### CITATION LIST

### PATENT LITERATURES

Patent Literature 1: Japanese Patent No. 6752811

### SUMMARY OF THE INVENTION

However, the problem of the conventional blood purification device is that flow rate drift (deviation of the detected flow rate) due to change over time, or malfunction, etc. causes a detection defect in each flow meter, which causes an error in the water removal amount.

Therefore, it is an object of the invention to provide a blood purification device and a method for determining detection defect in flow meter, which are capable of reducing water removal error due to detection defect in flow meter.

A blood purification device in an embodiment of the invention is a blood purification device to perform blood purification treatment on a patient using a blood purifier that purifies blood, the device comprising:
a dialysate supply flow path to supply dialysate to the blood purifier;
a waste liquid discharge flow path to discharge waste liquid from the blood purifier;
a first supply-side flow meter to detect a flow rate in the dialysate supply flow path;
a first discharge-side flow meter to detect a flow rate in the waste liquid discharge flow path;
a first water removal amount calculation unit that calculates a water removal amount based on the flow rate detected by the first supply-side flow meter and the flow rate detected by the first discharge-side flow meter;
a second water removal amount calculation unit that calculates a water removal amount based on a flow rate detected by a second supply-side flow meter provided to detect the flow rate in the dialysate supply flow path and a flow rate detected by a second discharge-side flow meter provided to detect the flow rate in the waste liquid discharge flow path, or calculates a theoretical water removal amount based on a target water removal rate and treatment time; and
a determination unit that determines a detection defect in the first supply-side flow meter and the first discharge-side flow meter based on a difference between the water removal amount calculated by the first water removal amount calculation unit and the water removal amount calculated by the second water removal amount calculation unit.

In addition, a method for determining detection defect in flow meter in an embodiment of the invention is a method for determining detection defect in flow meter to determine a detection defect in a first supply-side flow meter and a first discharge-side flow meter of a blood purification device that comprises a dialysate supply flow path to supply dialysate to a blood purifier that purifies blood, a waste liquid discharge flow path to discharge waste liquid from the blood purifier, the first supply-side flow meter to detect a flow rate in the dialysate supply flow path and the first discharge-side flow meter to detect a flow rate in the waste liquid discharge flow path, the method comprising:
performing a first water removal amount calculation step of calculating a water removal amount based on the flow rate detected by the first supply-side flow meter and the flow rate detected by the first discharge-side flow meter;
performing a second water removal amount calculation step of calculating a water removal amount based on a flow rate detected by a second supply-side flow meter provided to detect the flow rate in the dialysate supply flow path and a flow rate detected by a second discharge-side flow meter provided to detect the flow rate in the waste liquid discharge flow path, or calculating a theoretical water removal amount based on a target water removal rate and treatment time; and
performing a determination step of determining a detection defect in the first supply-side flow meter and the first discharge-side flow meter based on a difference between the water removal amount calculated in the first water removal amount calculation step and the water removal amount calculated in the second water removal amount calculation step.

### Advantageous Effects of the Invention

According to an embodiment of the invention, it is possible to reduce water removal error due to detection defect in flow meter.

### BRIEF DESCRIPTION OF DRAWINGS

Fig. 1 is a schematic structural diagram illustrating a structure of a blood purification device in an embodiment of the present invention.
Fig. 2 is a graph showing a control-side cumulative water removal amount, a protection-side cumulative water removal amount and a theoretical cumulative water removal amount to explain detection defect determination using these data.
Fig. 3 is a flowchart showing a detection defect monitoring operation.

### DETAILED DESCRIPTION OF THE INVENTION

A blood purification device and a method for determining detection defect in flow meter in an embodiment of the invention will be described below with reference to the appended drawings. This blood purification device is a medical device that performs dialysis treatment using a dialyzer to provide blood purification treatment on a patient, and is a so-called hemodialysis machine. In particular, this blood purification device employs a method for determining detection defect in flow meter, which can reduce water removal error caused by detection defect in flow meter.

### Configuration of Blood purification device

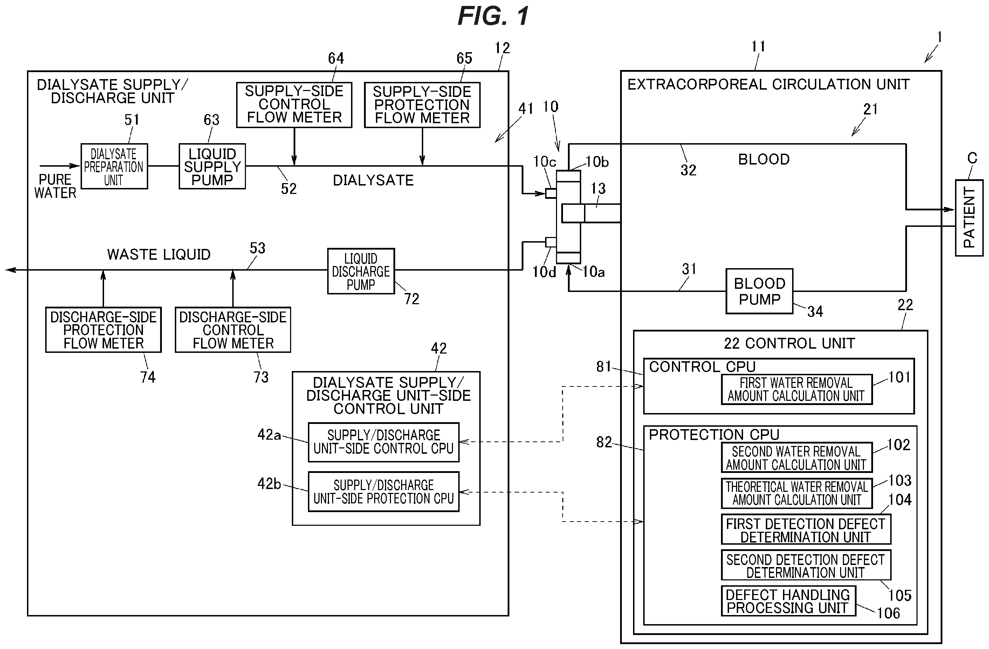

As shown in Fig. 1, a blood purification device 1 includes a dialyzer 10 that purifies blood of a patient C, an extracorporeal circulation unit 11 that circulates the blood of the patient C through the dialyzer 10, and a dialysate supply/discharge unit 12 that is connected to the dialyzer 10, supplies dialysate to the dialyzer 10 and discharges a waste liquid from the dialyzer 10. The extracorporeal circulation unit 11 and the dialysate supply/discharge unit 12 are configured as separate units, and the dialyzer 10 is removably attached to the extracorporeal circulation unit 11 through a fixing tool 13. The dialyzer 10 is an example of the blood purifier.

The dialysis device 10 has a blood purification membrane (a hollow-fiber hemodialysis membrane or hemodialysis filtration membrane, or a flat hemodialysis membrane or hemofiltration membrane) thereinside. The blood purifier 10 also has a blood inlet 10a to introduce blood and a blood outlet 10b to discharge the introduced blood, as well as a dialysate inlet 10c to introduce dialysate and a dialysate outlet 10d to discharge the introduced dialysate. In the dialysis device 10, blood is purified by bringing the blood into contact with dialysate through the blood purification membrane. In addition, in the dialyzer 10, removal of water from the blood of the patient C is made possible by controlling the flow rate of liquid supplied to the dialyzer 10 and the flow rate of liquid discharged from the dialyzer 10.

The extracorporeal circulation unit 11 has a blood circuit 21 that circulates the blood of the patient C through the dialyzer 10, and a control unit 22. The control unit 22 will be described later.

The blood circuit 21 has an artery-side blood flow path 31 that is connected to the blood inlet 10a of the dialyzer 10 and leads the blood collected from a blood vessel of the patient C to the dialyzer 10, a vein-side blood flow path 32 that is connected to the blood outlet 10b of the dialyzer 10 and returns the blood discharged from the dialyzer 10 to the blood vessel of the patient C, and a blood pump 34 arranged on the artery-side blood flow path 31 to circulate the blood. The blood from the patient C is led to the dialyzer 10 through the artery-side blood flow path 31 by driving the blood pump 34, and the blood is purified by the dialyzer 10 and is then returned to the patient C through the vein-side blood flow path 32. The blood of the patient C is thereby purified.

### Configuration of Dialysate supply/discharge unit

The dialysate supply/discharge unit 12 has a dialysate circuit 41 that supplies dialysate to the dialyzer 10 and also discharges a waste liquid from the dialyzer 10, and a dialysate supply/discharge unit-side control unit 42.

The dialysate circuit 41 has a dialysate preparation unit 51 that refines dialysate, a dialysate supply flow path 52 that is connected to the dialysate inlet 10c of the dialyzer 10 and supplies the dialysate refined by the dialysate preparation unit 51 to the dialyzer 10, and a waste liquid discharge flow path 53 that is connected to the dialysate outlet 10d of the dialyzer 10 and collects and discharges the waste liquid from the dialyzer 10.

The dialysate preparation unit 51 prepares dialysate from pure water supplied thereto and a dialysate agent made of a concentrated solution or powder. The pure water supplied to the dialysate preparation unit 51 may be supplied from a pure water production unit mounted on the dialysate supply/discharge unit 12, or may be supplied from a pure water production device provided outside the dialysate supply/discharge unit 12. In this regard, the dialysate preparation unit 51 can be omitted, and the configuration may be such that, e.g., dialysate is supplied to the dialysate supply/discharge unit 12 from an external dialysate supply device, etc.

From the upstream side, a liquid supply pump 63, a supply-side control flow meter 64 and a supply-side protection flow meter 65 are arranged on the dialysate supply flow path 52. The supply-side control flow meter 64 is an example of the first supply-side flow meter, and the supply-side protection flow meter 65 is an example of the second supply-side flow meter.

The liquid supply pump 63 is a liquid feed pump that feeds the dialysate in the dialysate supply flow path 52. The dialysate is supplied to the dialyzer 10 by driving the liquid supply pump 63.

The supply-side control flow meter 64 and the supply-side protection flow meter 65 are flow meters that are arranged on the downstream side of the liquid supply pump 63 and detect the flow rate in the dialysate supply flow path 52 (i.e., the flow rate of the liquid supplied to the dialyzer 10). The supply-side control flow meter 64 is a flow meter for control that detects the flow rate as a controlled variable in feedback control of the liquid supply pump 63. On the other hand, the supply-side protection flow meter 65 is a flow meter for protection to ensure that the supply-side control flow meter 64 and its detection value are normal. The term "flow rate" here refers to the amount of fluid moved per unit time. In addition, the flow rate detected by the supply-side control flow meter 64 and the supply-side protection flow meter 65 can also be said to be the flow rate of the liquid supply pump 63.

From the upstream side, a liquid discharge pump 72, a discharge-side control flow meter 73 and a discharge-side protection flow meter 74 are arranged on the waste liquid discharge flow path 53. The discharge-side control flow meter 73 is an example of the first discharge-side flow meter and the discharge-side protection flow meter 74 is an example of the second discharge-side flow meter.

The liquid discharge pump 72 is a liquid feed pump that feeds the waste liquid in the waste liquid discharge flow path 53. The waste liquid from the dialyzer 10 is discharged by driving the liquid discharge pump 72. The flow rate of the liquid supply pump 63 and the flow rate of the liquid discharge pump 72 are controlled by controlling the liquid supply pump 63 and the liquid discharge pump 72, and the amount of water removed from the blood in the dialyzer 10 is thereby controlled.

The discharge-side control flow meter 73 and the discharge-side protection flow meter 74 are flow meters that are arranged on the downstream side of the liquid discharge pump 72 and detect the flow rate in the waste liquid discharge flow path 53 (i.e., the flow rate of the waste liquid from the dialyzer 10). The discharge-side control flow meter 73 is a flow meter for control that detects the flow rate as a controlled variable in feedback control of the liquid discharge pump 72. On the other hand, the discharge-side protection flow meter 74 is a flow meter for protection to ensure that the discharge-side control flow meter 73 and its detection value are normal. In this regard, the flow rate detected by the discharge-side control flow meter 73 and the discharge-side protection flow meter 74 can also be said to be the flow rate of the liquid discharge pump 72.

The dialysate supply/discharge unit-side control unit 42 communicates with the control unit 22 of the extracorporeal circulation unit 11, and controls the liquid supply pump 63 and the liquid discharge pump 72 according to commands from the control unit 22. The dialysate supply/discharge unit-side control unit 42 has a supply/discharge unit-side control CPU 42a to control the liquid supply pump 63 and the liquid discharge pump 72, and a supply/discharge unit-side protection CPU 42b to ensure operation of the supply/discharge unit-side control CPU 42a. Each of the supply/discharge unit-side control CPU 42a and the supply/discharge unit-side protection CPU 42b is realized by appropriately combining an arithmetic element such as CPU, a memory, software, interface and a communication unit, etc. That is, a control CPU 81 and a protection CPU 82 are configured as separate hardware and can operate independently.

### Description of Control unit and its control

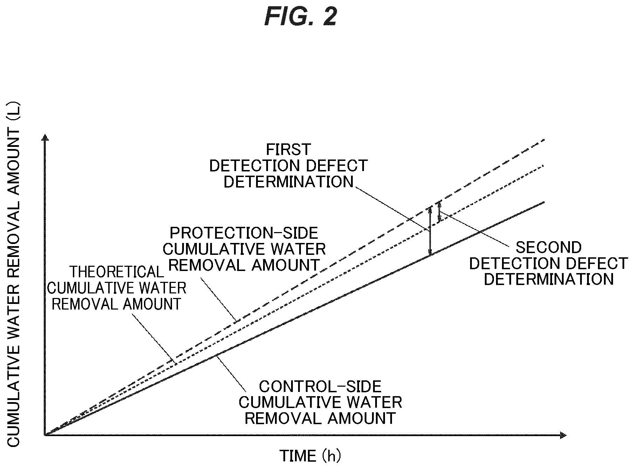

The control unit 22 and the control by the control unit 22 will now be described in reference to Figs. 1 and 2. As shown in Fig. 1, the control unit 22 has the control CPU 81 (a so-called main control unit) that controls each unit of the blood purification device 1, and the protection CPU 82 (a so-called sub-control unit) to ensure operation of the control CPU 81. Each of the control CPU 81 and the protection CPU 82 is realized by appropriately combining an arithmetic element such as CPU, a memory, software, interface and a communication unit, etc. That is, the control CPU 81 and the protection CPU 82 are configured as separate hardware and can operate independently.

The control CPU 81 controls drive of the blood pump 34. In addition, the control CPU 81 communicates with the supply/discharge unit-side control CPU 42a, receives detection values of the supply-side control flow meter 64 and the discharge-side control flow meter 73 through the supply/discharge unit-side control CPU 42a, and controls the liquid supply pump 63 and the liquid discharge pump 72. On the other hand, the protection CPU 82 communicates with the supply/discharge unit-side protection CPU 42b and receives detection values of the supply-side protection flow meter 65 and the discharge-side protection flow meter 74 through the supply/discharge unit-side protection CPU 42b.

The control CPU 81 drives the blood pump 34, the liquid supply pump 63 and the liquid discharge pump 72 to perform dialysis treatment. That is, during the dialysis treatment, the blood pump 34 is driven to circulate blood through the dialyzer 10 and the liquid supply pump 63 is driven to supply dialysate to the dialyzer 10, while the liquid discharge pump 72 is driven to discharge the waste liquid from dialyzer 10. During this dialysis treatment operation, the flow rate of liquid supplied to the dialyzer 10 (the flow rate in the dialysate supply flow path 52) and the flow rate of liquid discharged from the dialyzer 10 (the flow rate in the waste liquid discharge flow path 53) are controlled by controlling the liquid supply pump 63 and the liquid discharge pump 72, and the amount of water removed from the blood of the patient C is thereby controlled. In the present embodiment, the liquid supply pump 63 is driven to achieve a target flow rate under feedback control using the detection value of the supply-side control flow meter 64 as the controlled variable, and the liquid discharge pump 74 is driven to achieve a target flow rate under feedback control using the detection value of the discharge-side control flow meter 73 as the controlled variable.

In the meantime, during dialysis treatment operation, flow rate drift (deviation of the detected flow rate) due to change over time, or malfunction, etc. may cause a detection defect in the control flow meters 64, 73. When a detection defect in the control flow meters 64, 73 occurs, this detection defect affects the flow rate control of the dialysate supply flow path 52 and the waste liquid discharge flow path 53, resulting in that an error in the amount of water removed from the blood occurs. To address this, the blood purification device 1 in the present embodiment has a configuration to monitor a detection defect in the control flow meters 64, 73 during the dialysis treatment operation. As shown in Fig. 1, in the blood purification device 1, as a configuration to monitor a detection defect in the control flow meters 64, 73, the control CPU 81 constitutes a first water removal amount calculation unit 101 and the protection CPU 82 constitutes a second water removal amount calculation unit 102, a theoretical water removal amount calculation unit 103, a first detection defect determination unit 104, a second detection defect determination unit 105 and a defect handling processing unit 106. The theoretical water removal amount calculation unit 103 is an example of the third water removal amount calculation unit, and the first detection defect determination unit 104 and the second detection defect determination unit 105 are examples of the determination unit.

Based on the flow rate in the dialysate supply flow path 52 detected by the supply-side control flow meter 64 and the flow rate in the waste liquid discharge flow path 53 detected by the discharge-side control flow meter 73, the first water removal amount calculation unit 101 calculates a cumulative water removal amount (hereinafter, referred to as the "control-side cumulative water removal amount"). **In** particular, the flow rate detected by the discharge-side control flow meter 73 is subtracted from the flow rate detected by the supply-side control flow meter 64 to calculate the water removal amount (the flow rate difference) per unit time and the calculated water removal amount is added up, thereby calculating the control-side cumulative water removal amount.

Based on the flow rate in the dialysate supply flow path 52 detected by the supply-side protection flow meter 65 and the flow rate in the waste liquid discharge flow path 53 detected by the discharge-side protection flow meter 74, the second water removal amount calculation unit 102 calculates a cumulative water removal amount (hereinafter, referred to as the "protection-side cumulative water removal amount"). In particular, the flow rate detected by the discharge-side protection flow meter 74 is subtracted from the flow rate detected by the supply-side protection flow meter 65 to calculate the water removal amount per unit time and the calculated water removal amount is added up, thereby calculating the protection-side cumulative water removal amount.

The theoretical water removal amount calculation unit 103 calculates a water removal amount in theory (hereinafter, referred to as the "theoretical cumulative water removal amount") based on a target water removal rate and treatment time. In particular, theoretical cumulative water removal amount is calculated by multiplying the target water removal rate by the treatment time.

**In** this regard, the cumulative water removal amount calculated by the first water removal amount calculation unit 101, the second water removal amount calculation unit 102 and the theoretical water removal amount calculation unit 103 may be a cumulative water removal amount from the start of the treatment, which is calculated by adding up (accumulating) the water removal amount from the start of the treatment, or it may be a cumulative water removal amount obtained in such a manner that the overall treatment time is divided into plural sections (e.g., sections at certain intervals) and the water removal amount from the start of each section is added up (accumulated). Alternatively, it may be a cumulative water removal amount for a predetermined period of time, which is calculated by adding up the water removal amount from a predetermined time before the current time (e.g., one hour before) to the current time.

The first detection defect determination unit 104 performs a first detection defect determination for the control flow meters 64, 73 based on a difference between the control-side cumulative water removal amount calculated by the first water removal amount calculation unit 101 and the protection-side cumulative water removal amount calculated by the second water removal amount calculation unit 102, as shown in Fig. 2. In particular, the first detection defect determination unit 104 calculates a difference between the control-side cumulative water removal amount calculated by the first water removal amount calculation unit 101 and the protection-side cumulative water removal amount calculated by the second water removal amount calculation unit 102 and, when the difference is determined to be not less than a first threshold value, determines that there is a significant detection defect in the control flow meters 64, 73. Then, when the difference is determined to be not less than a second threshold value, which is smaller than the first threshold value, and less than the first threshold value, it is determined that there is a minor detection defect in the control flow meters 64, 73. On the other hand, when the difference is determined to be less than the second threshold value, it is determined that there is no detection defect in the control flow meters 64, 73.

The second detection defect determination unit 105 performs a second detection defect determination for the control flow meters 64, 73 based on a difference between the protection-side cumulative water removal amount calculated by the second water removal amount calculation unit 102 and the theoretical cumulative water removal amount calculated by the theoretical water removal amount calculation unit103, as shown in Fig. 2. In particular, the second detection defect determination unit 105 calculates a difference between the protection-side cumulative water removal amount calculated by the second water removal amount calculation unit 102 and the theoretical cumulative water removal amount calculated by the theoretical water removal amount calculation unit103 and, when the difference is determined to be not less than a threshold value, determines that there is a significant detection defect in the control flow meters 64, 73. On the other hand, when the difference is determined to be less than the threshold value, it is determined that there is no detection defect in the control flow meters 64, 73. In this regard, the threshold value used by the second detection defect determination unit 105 is preferably the same value as the first threshold value used by the first detection defect determination unit 104, but the threshold value used by the second detection defect determination unit 105 may be a value different from the first threshold value or the second threshold value used by the first detection defect determination unit 104.

The defect handling processing unit 106 executes a handling process based on the determination results of the first detection defect determination unit 104 and the second detection defect determination unit 105. In particular, when the first detection defect determination unit 104 or the second detection defect determination unit 105 determines that there is a significant detection defect in the control flow meters 64, 73, an alarm is output and the dialysis treatment operation is stopped. On the other hand, when the first detection defect determination unit 104 determines that there is a minor detection defect in the control flow meters 64, 73, the control flow meters 64, 73 are calibrated and the dialysis treatment operation continues. In calibrating the control flow meters 64, 73, for example, a correction value of the detected flow rate is corrected based on the difference between the control-side cumulative water removal amount and the protection-side cumulative water removal amount calculated by the first detection defect determination unit 104.

### Description of Detection defect monitoring operation

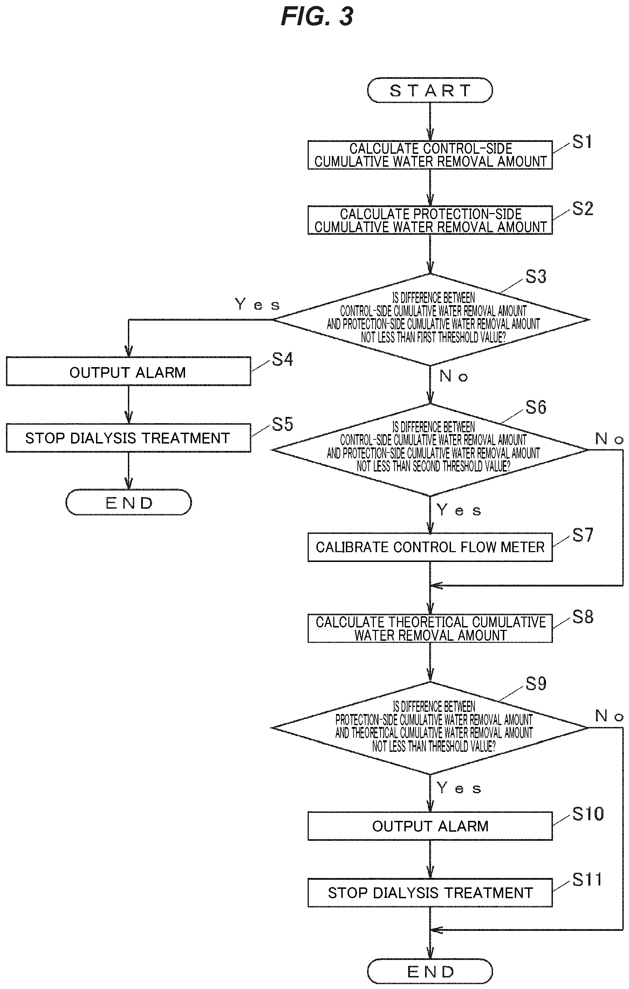

A detection defect monitoring operation by the blood purification device 1 will now be described in reference to Fig. 3. This detection defect monitoring operation is executed by the control unit 22 every second during the dialysis treatment operation, and is an operation to determine a detection defect in the control flow meters 64, 73. The detection defect monitoring operation is an example of the method for determining detection defect in flow meter.

As shown in Fig. 3, in the detection defect monitoring operation, first, the control-side cumulative water removal amount is calculated by the first water removal amount calculation unit 101 (S1) (the first water removal amount calculation step). That is, the current water removal amount is calculated by subtracting the flow rate in the waste liquid discharge flow path 53 detected by the discharge-side control flow meter 73 from the flow rate in the dialysate supply flow path 52 detected by the supply-side control flow meter 64. Then, the control-side cumulative water removal amount is calculated by adding the calculated water removal amount to the water removal amount calculated by the first water removal amount calculation unit 101 up to the last detection defect monitoring operation.

After that, the protection-side cumulative water removal amount is calculated by the second water removal amount calculation unit 102 (S2) (the second water removal amount calculation step). That is, the current water removal amount is calculated by subtracting the flow rate in the waste liquid discharge flow path 53 detected by the discharge-side protection flow meter 74 from the flow rate in the dialysate supply flow path 52 detected by the supply-side protection flow meter 65. Then, the control-side cumulative water removal amount is calculated by adding the calculated water removal amount to the water removal amount calculated by the second water removal amount calculation unit 102 up to the last detection defect monitoring operation.

After calculating the control-side cumulative water removal amount and the protection-side cumulative water removal amount, the first detection defect determination is performed by the first detection defect determination unit 104 based on the calculated control-side cumulative water removal amount and protection-side cumulative water removal amount (the determination step). In particular, first, whether or not a difference between the control-side cumulative water removal amount and the protection-side cumulative water removal amount is not less than the first threshold value is determined (S3). When it is determined that the difference between the control-side cumulative water removal amount and the protection-side cumulative water removal amount is not less than the first threshold value (S3: Yes), it is determined that there is a significant detection defect in the control flow meters 64, 73, the defect handling processing unit 106 then outputs an alarm (S4) and stops the dialysis treatment operation (S5), and this detection defect monitoring operation ends.

On the other hand, when it is determined that the difference between the control-side cumulative water removal amount and the protection-side cumulative water removal amount is less than the first threshold value (S3: No), whether or not the difference between the control-side cumulative water removal amount and the protection-side cumulative water removal amount is not less than the second threshold value is determined (S6). When it is determined that the difference between the control-side cumulative water removal amount and the protection-side cumulative water removal amount is not less than the second threshold value (S6: Yes), it is determined that there is a minor detection defect in the control flow meters 64, 73 and the defect handling processing unit 106 calibrates the control flow meters 64, 73 (S7). On the other hand, when it is determined that the difference between the control-side cumulative water removal amount and the protection-side cumulative water removal amount is less than the second threshold value (S6: No), the control flow meters 64, 73 are not calibrated.

After that, the theoretical cumulative water removal amount is calculated by the theoretical water removal amount calculation unit 103 (S8). That is, the theoretical cumulative water removal amount is calculated by multiplying the target water removal rate by the treatment time.

After calculating the theoretical cumulative water removal amount, the second detection defect determination is performed by the second detection defect determination unit 105 based on the calculated protection-side cumulative water removal amount and theoretical cumulative water removal amount. In particular, whether or not a difference between the protection-side cumulative water removal amount and the theoretical cumulative water removal amount is not less than the threshold value is determined (S9). When it is determined that the difference between the protection-side cumulative water removal amount and the theoretical cumulative water removal amount is not less than the threshold value (S9: Yes), it is determined that there is a significant detection defect in the control flow meters 64, 73, the defect handling processing unit 106 then outputs an alarm (S10) and stops the dialysis treatment operation (S11), and this detection defect monitoring operation ends. On the other hand, when it is determined that the difference between the protection-side cumulative water removal amount and the theoretical cumulative water removal amount is less than the threshold value (S9: No), it is determined that there is no detection defect in the control flow meters 64, 73, and this detection defect monitoring operation ends.

### Functions and Effects of the embodiment

In the configuration of the embodiment described above, since a detection defect in the control flow meters 64, 73 is determined based on the difference between the cumulative water removal amounts, it is possible to appropriately determine the detection defect associated with water removal error. It is thereby possible to reduce water removal error due to the detection defect in the control flow meters 64 and 73.

In addition, by performing the detection defect determination based on the difference between the protection-side cumulative water removal amount and the theoretical cumulative water removal amount (the second detection defect determination) in addition to the detection defect determination based on the difference between the control-side cumulative water removal amount and the protection-side cumulative water removal amount (the first detection defect determination), the detection defect in the control flow meters 64, 73 can be accurately determined even in the event where similar detection defects occur in the control flow meters 64, 73 and the protection flow meters 65, 74.

In addition, the protection CPU 82 is provided in addition to the control CPU 81 and the protection CPU 82 receives the detection values of the supply-side protection flow meter 65 and the discharge-side protection flow meter 74 and also constitutes the second water removal amount calculation unit 102 and the first detection defect determination unit 104. Therefore, even if the cause of the detection defect lies in the control CPU 81, it is possible to address this.

### Modifications

Although the embodiment of the invention has been described, the invention according to claims is not to be limited to the embodiment described above. Further, please note that not all combinations of the features described in the embodiment are necessary to solve the problem of the invention.

For example, the configuration in the embodiment described above is such that the second water removal amount calculation unit 102 calculates the protection-side cumulative water removal amount based on the flow rate detected by the supply-side protection flow meter 65 and the flow rate detected by the discharge-side protection flow meter 74 and the first detection defect determination unit 104 performs the detection defect determination based on the difference between the control-side cumulative water removal amount and the protection-side cumulative water removal amount, but the configuration is not limited thereto. That is, the configuration may be such that the second water removal amount calculation unit 102 calculates the theoretical cumulative water removal amount based on the target water removal rate and the treatment time and the first detection defect determination unit 104 performs the detection defect determination based on a difference between the control-side cumulative water removal amount and the theoretical cumulative water removal amount. In such a case, the theoretical cumulative water removal amount is calculated based on the target water removal rate and the treatment time in the second water removal amount calculation step (S2).

In addition, the configuration in the embodiment described above is such that the first detection defect determination unit 104 and the second detection defect determination unit 105 perform the detection detect determination based on a difference between two cumulative water removal amounts, but the configuration is not limited thereto as long as the detection detect determination is performed based on a difference between two cumulative water removal amounts. In other words, it may be configured to perform the detection detect determination based on a value obtained by subtracting one cumulative water removal amount from the other cumulative water removal amount.

In addition, the configuration in the embodiment described above is such that the first water removal amount calculation unit 101, the second water removal amount calculation unit 102 and the theoretical water removal amount calculation unit 103 calculate the cumulative water removal amounts and the first detection defect determination unit 104 and the second detection defect determination unit 105 perform the detection defect determination based on the difference between the cumulative water removal amounts, but the configuration is not limited thereto. That is, the configuration may be such that the first water removal amount calculation unit 101, the second water removal amount calculation unit 102 and the theoretical water removal amount calculation unit 103 calculate water removal amounts (water removal rates) per unit time, and the first detection defect determination unit 104 and the second detection defect determination unit 105 perform the detection defect determination based on the difference between the water removal amounts per unit time.

In addition, the configuration in the embodiment described above is such that the calibration of the control flow meters 64, 73 is performed only when it is determined that there is a minor detection defect in the control flow meters 64, 73, but the calibration of the control flow meters 64, 73 can be performed at any time. It may be configured to calibrate the control flow meters 64 and 73, e.g., when the dialysis treatment operation is started or when the dialysis treatment operation is stopped with an alarm. This configuration also makes it possible to address cases where a user ignores the warning and resumes the treatment. In addition, it may be configured to calibrate, e.g., before the detection defect monitoring operation or before determining whether or not there is a minor detection defect (S6).

In addition, the configuration in the embodiment described above is such that in the detection defect monitoring operation, whether or not there is a minor detection defect in the control flow meters 64, 73 (S6) is determined and the control flow meters 64, 73 are calibrated when it is determined that there is a minor detection defect in the control flow meters 64, 73 (S7), but the configuration may be such that the determination of whether or not there is a minor detection defect in the control flow meters 64, 73 (S6) and the calibration of the control flow meters 64, 73 (S7) are omitted in the detection defect monitoring operation.

In addition, the configuration in the embodiment described above is such that the supply-side control flow meter 64 and the discharge-side control flow meter 73 are used as the first supply-side flow meter and the first discharge-side flow meter and the detection defect in the control flow meters 64, 73 is determined based on the difference between the water removal amounts, but the configuration is not limited thereto. That is, the configuration may be such that the supply-side protection flow meter 65 and the discharge-side protection flow meter 74 are used as the first supply-side flow meter and the first discharge-side flow meter and the detection defect in the protection flow meters 65, 74 is determined based on the difference between the water removal amounts.

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.
(1) A blood purification device 1 to perform blood purification treatment on a patient C using a blood purifier 10 that purifies blood, the blood purification device 1 comprising: a dialysate supply flow path 52 to supply dialysate to the blood purifier 10; a waste liquid discharge flow path 53 to discharge waste liquid from the blood purifier 10; a first supply-side flow meter 64 to detect a flow rate in the dialysate supply flow path 52; a first discharge-side flow meter 73 to detect a flow rate in the waste liquid discharge flow path 53; a first water removal amount calculation unit 101 that calculates a water removal amount based on the flow rate detected by the first supply-side flow meter 64 and the flow rate detected by the first discharge-side flow meter 73; a second water removal amount calculation unit 102 that calculates a water removal amount based on a flow rate detected by a second supply-side flow meter 65 provided to detect the flow rate in the dialysate supply flow path 52 and a flow rate detected by a second discharge-side flow meter 74 provided to detect the flow rate in the waste liquid discharge flow path 53, or calculates a theoretical water removal amount based on a target water removal rate and treatment time; and a determination unit 104, 105 that determines a detection defect in the first supply-side flow meter 64 and the first discharge-side flow meter 73 based on a difference between the water removal amount calculated by the first water removal amount calculation unit 101 and the water removal amount calculated by the second water removal amount calculation unit 102.
   It is thereby possible to reduce water removal error due to the detection defect in the flow meter.
(2) The blood purification device 1 as defined by (1), further comprising: a third water removal amount calculation unit 103 that calculates a theoretical water removal amount based on a target water removal rate and treatment time, wherein the second water removal amount calculation unit 102 calculates a water removal amount based on the flow rate detected by the second supply-side flow meter 65 and the flow rate detected by the second discharge-side flow meter 74, and wherein the determination unit 104, 105 determines a detection defect in the first supply-side flow meter 64 and the first discharge-side flow meter 73 based on a difference between the water removal amount calculated by the second water removal amount calculation unit 102 and the water removal amount calculated by the third water removal amount calculation unit 103. As a result, even when similar detection defects occur in the first supply-side flow meter and the first discharge-side flow meter and in the second supply-side flow meter and the second discharge-side flow meter, it is possible to accurately determine the detection defect in the first supply-side flow meter and the first discharge-side flow meter.
(3) The blood purification device 1 as defined by (1) or (2), further comprising: a liquid supply pump 63 that is arranged on the dialysate supply flow path 52 and feeds the dialysate; and a liquid discharge pump 72 that is arranged on the waste liquid discharge flow path 53 and feeds the waste liquid, wherein the first supply-side flow meter 64 is a flow meter for control that detects the flow rate as a controlled variable in feedback control of the liquid supply pump 63, wherein the second supply-side flow meter 65 is a flow meter for protection to ensure a detection value of the first supply-side flow meter 64, wherein the first discharge-side flow meter 73 is a flow meter for control that detects the flow rate as a controlled variable in feedback control of the liquid discharge pump 72, and wherein the second discharge-side flow meter 74 is a flow meter for protection to ensure a detection value of the first discharge-side flow meter 73.
   It is thereby possible to reduce water removal error due to the detection defect in the control flow meter.
(4) The blood purification device 1 as defined by (3), further comprising: a control CPU 81 that controls the liquid supply pump 63 and the liquid discharge pump 72; and a protection CPU 82 to ensure operation of the control CPU 81, wherein the control CPU 81 receives detection values of the first supply-side flow meter 64 and the first discharge-side flow meter 73 and constitutes the first water removal amount calculation unit 101, and wherein the protection CPU 82 receives detection values of the second supply-side flow meter 65 and the second discharge-side flow meter 74 and constitutes the second water removal amount calculation unit 102 and the determination unit 104, 105.
   As a result, when the cause of the detection defect lies in the control CPU, it is possible to address this.
(5) A method for determining detection defect in flow meter 74, 64 to determine a detection defect in a first supply-side flow meter 64 and a first discharge-side flow meter 65 of a blood purification device 1 that comprises a dialysate supply flow path 52 to supply dialysate to a blood purifier 10 that purifies blood, a waste liquid discharge flow path 53 to discharge waste liquid from the blood purifier 10, the first supply-side flow meter 64 to detect a flow rate in the dialysate supply flow path 52 and the first discharge-side flow meter 65 to detect a flow rate in the waste liquid discharge flow path 53, the method for determining detection defect in flow meter 74, 64 comprising: performing a first water removal amount calculation step S1 of calculating a water removal amount based on the flow rate detected by the first supply-side flow meter 64 and the flow rate detected by the first discharge-side flow meter 73; performing a second water removal amount calculation step S2 of calculating a water removal amount based on a flow rate detected by a second supply-side flow meter 65 provided to detect the flow rate in the dialysate supply flow path 52 and a flow rate detected by a second discharge-side flow meter 74 provided to detect the flow rate in the waste liquid discharge flow path 53, or calculating a theoretical water removal amount based on a target water removal rate and treatment time; and performing a determination step S3, S6 of determining a detection defect in the first supply-side flow meter 64 and the first discharge-side flow meter 73 based on a difference between the water removal amount calculated in the first water removal amount calculation step S1 and the water removal amount calculated in the second water removal amount calculation step S2.

It is thereby possible to reduce water removal error due to the detection defect in the flow meter.

### REFERENCE SIGNS LIST

1: blood purification device, 10: dialyzer, 52: dialysate supply flow path, 53: waste liquid discharge flow path, 63: liquid supply pump, 64: supply-side control flow meter, 65: supply-side protection flow meter, 72: liquid discharge pump, 73: discharge-side control flow meter, 74: discharge-side protection flow meter, 81: control CPU, 82: protection CPU, 101: first water removal amount calculation unit, 102: second water removal amount calculation unit, 103: theoretical water removal amount calculation unit, 104: first detection defect determination unit, 105: second detection defect determination unit, C: patient

## Claims

1. A blood purification device to perform blood purification treatment on a patient using a blood purifier that purifies blood, the blood purification device comprising:
a dialysate supply flow path to supply dialysate to the blood purifier;
a waste liquid discharge flow path to discharge waste liquid from the blood purifier;
a first supply-side flow meter to detect a flow rate in the dialysate supply flow path;
a first discharge-side flow meter to detect a flow rate in the waste liquid discharge flow path;
a first water removal amount calculation unit that calculates a water removal amount based on the flow rate detected by the first supply-side flow meter and the flow rate detected by the first discharge-side flow meter;
a second water removal amount calculation unit that calculates a water removal amount based on a flow rate detected by a second supply-side flow meter provided to detect the flow rate in the dialysate supply flow path and a flow rate detected by a second discharge-side flow meter provided to detect the flow rate in the waste liquid discharge flow path, or calculates a theoretical water removal amount based on a target water removal rate and treatment time; and
a determination unit that determines a detection defect in the first supply-side flow meter and the first discharge-side flow meter based on a difference between the water removal amount calculated by the first water removal amount calculation unit and the water removal amount calculated by the second water removal amount calculation unit.

2. The blood purification device according to claim 1, further comprising:
a third water removal amount calculation unit that calculates a theoretical water removal amount based on a target water removal rate and treatment time,
wherein the second water removal amount calculation unit calculates a water removal amount based on the flow rate detected by the second supply-side flow meter and the flow rate detected by the second discharge-side flow meter, and wherein the determination unit determines a detection defect in the first supply-side flow meter and the first discharge-side flow meter based on a difference between the water removal amount calculated by the second water removal amount calculation unit and the water removal amount calculated by the third water removal amount calculation unit.

3. The blood purification device according to claim 1 or 2, further comprising:
a liquid supply pump that is arranged on the dialysate supply flow path and feeds the dialysate; and
a liquid discharge pump that is arranged on the waste liquid discharge flow path and feeds the waste liquid,
wherein the first supply-side flow meter is a flow meter for control that detects the flow rate as a controlled variable in feedback control of the liquid supply pump, wherein the second supply-side flow meter is a flow meter for protection to ensure a detection value of the first supply-side flow meter, wherein the first discharge-side flow meter is a flow meter for control that detects the flow rate as a controlled variable in feedback control of the liquid discharge pump, and wherein the second discharge-side flow meter is a flow meter for protection to ensure a detection value of the first discharge-side flow meter.

4. The blood purification device according to claim 3, further comprising:
a control CPU that controls the liquid supply pump and the liquid discharge pump; and
a protection CPU to ensure operation of the control CPU,
wherein the control CPU receives detection values of the first supply-side flow meter and the first discharge-side flow meter and constitutes the first water removal amount calculation unit, and wherein the protection CPU receives detection values of the second supply-side flow meter and the second discharge-side flow meter and constitutes the second water removal amount calculation unit and the determination unit.

5. A method for determining detection defect in flow meter to determine a detection defect in a first supply-side flow meter and a first discharge-side flow meter of a blood purification device that comprises a dialysate supply flow path to supply dialysate to a blood purifier that purifies blood, a waste liquid discharge flow path to discharge waste liquid from the blood purifier, the first supply-side flow meter to detect a flow rate in the dialysate supply flow path and the first discharge-side flow meter to detect a flow rate in the waste liquid discharge flow path, the method for determining detection defect in flow meter comprising:
performing a first water removal amount calculation step of calculating a water removal amount based on the flow rate detected by the first supply-side flow meter and the flow rate detected by the first discharge-side flow meter;
performing a second water removal amount calculation step of calculating a water removal amount based on a flow rate detected by a second supply-side flow meter provided to detect the flow rate in the dialysate supply flow path and a flow rate detected by a second discharge-side flow meter provided to detect the flow rate in the waste liquid discharge flow path, or calculating a theoretical water removal amount based on a target water removal rate and treatment time; and
performing a determination step of determining a detection defect in the first supply-side flow meter and the first discharge-side flow meter based on a difference between the water removal amount calculated in the first water removal amount calculation step and the water removal amount calculated in the second water removal amount calculation step.
